# FASCICULE DE BREVET EUROPEEN

(11) **EP 3 049 120 B1**
(45) Date de publication et mention de la délivrance du brevet: **31.10.2018**
(21) Numéro de dépôt: 13782747.3
(22) Date de dépôt: 24.09.2013
(51) Int. Cl.: A61L 15/58, A61L 24/04

(54) **PROCEDE DE REALISATION D'UN MATERIAU ADHESIF POUR APPLICATION MEDICALE**
VERFAHREN ZUR HERSTELLUNG EINES KLEBSTOFFS FÜR MEDIZINISCHE ANWENDUNGEN
METHOD FOR PRODUCING AN ADHESIVE MATERIAL FOR MEDICAL APPLICATION

(43) Date de publication de la demande: 03.08.2016
(73) Titulaire: Zodiac Coating, 78370 Plaisir (FR)
(72) Inventeur: GUILLERMIN, Mélanie, 38230 Charvieu Chavagneux (FR); GUILLO, Jean-Roger, 56340 Carnac (FR); LECOEUVRE, Jean-François, 42290 Sorbiers (FR)
(74) Mandataire: Zimmermann, Alain
(86) Numéro de dépôt international: PCT/FR2013/052234
(87) Numéro de publication internationale: WO 2015/044532

(56) Documents cités:
- EP-A1- 2 561 844
- EP-A1- 2 561 896
- WO-A1-2005/102403
- FR-A1- 2 971 971
- David C Gantner ET AL: "Soft Skin Adhesive Gels and Liners: New Formulating Options for Tailored Solutions", , 1 janvier 2007 (2007-01-01), XP055124225, Extrait de l'Internet: URL:http://www.dowcorning.com/content/publ ishedlit/52-1113.pdf [extrait le 2014-06-19]

## Description

La présente invention se rapporte à un procédé de réalisation d'un matériau adhésif pour application médicale tel qu'un pansement, et le matériau ainsi réalisé.

Il existe actuellement différents types de matériaux adhésifs à usage de traitement des plaies (composants de pansements, films adhésifs de protection) et/ou à usage de maintien de compresses ou de dispositifs médicaux (rubans adhésifs, patchs de maintien) destinés au contact atraumatique de la peau saine et de la plaie.

Habituellement, de tels matériaux adhésifs sont obtenus par différents procédés visant à déposer un adhésif sur un substrat de destination.

Ainsi, il est par exemple connu un procédé d'enduction directe de l'adhésif sur le substrat de destination, suivi d'un traitement thermique de l'adhésif (séchage ou réticulation) et de la pose d'un protecteur (liner) sur la face adhésive du matériau obtenu. Usuellement, le substrat de destination peut faire l'objet d'une enduction d'un primaire d'adhérisation ou d'un traitement corona destiné à favoriser l'adhésion entre substrat et adhésif.

Cependant, ce procédé ne peut s'appliquer qu'aux substrats :
- compatibles thermiquement (absence de fusion, ramollissement et de retrait), et
- présentant une faible porosité s'opposant au traversement du substrat par le silicone lors de l'enduction.

Ce procédé nécessite souvent une étape préalable, donc coûteuse, de dépôt d'une couche primaire d'adhérisation. Il en résulte que ce procédé est très limitatif en termes de matériaux réalisables et peut présenter un surcoût de réalisation.

Selon une variante de réalisation connue, l'adhésif est enduit sur un film traité anti-adhérent pour cet adhésif, puis une opération de doublage est suivie d'un laminage du substrat de destination sur l'adhésif avant de réaliser un traitement thermique de l'ensemble (séchage ou réticulation).

Néanmoins, ce procédé nécessite des substrats de destination :
- compatibles thermiquement (absence de fusion, ramollissement et/ou retrait) avec un passage dans l'étape de réticulation du silicone,
- présentant une porosité et/ou une capillarité limitée(s) s'opposant au traversement et/ou l'absorption de l'adhésif dans et/ou par le substrat de destination durant le laps de temps situé entre le doublage du substrat sur l'adhésif à l'état liquide et la réticulation de l'adhésif, et
- présentant une rugosité ou une surface fibreuse permettant l'ancrage de l'adhésif dans le cas où une étape préalable de dépose d'un primaire est à éviter.

Ce procédé nécessite souvent une étape préalable, donc coûteuse, de dépôt d'une couche primaire d'adhérisation car l'adhésion entre le silicone et le substrat ne peut suffire à l'application, même avec traitement corona du substrat avant enduction.

Il en résulte que ce procédé est moins limitatif en termes de matériaux réalisables que le précédent, mais il présente lui aussi un surcoût de réalisation.

Selon un dernier mode de réalisation, l'adhésif est enduit sur un film traité anti-adhérent pour cet adhésif, l'adhésif est traité thermiquement (séchage ou réticulation), puis une opération de doublage est effectuée, suivie par un laminage du substrat de destination sur la face adhésive. En règle générale, le substrat de destination fait l'objet d'un traitement corona destiné à favoriser l'adhésion entre substrat et adhésif.

Ce procédé devrait permettre d'ouvrir les possibilités aux substrats de destination sensibles thermiquement (sujets à la fusion au ramollissement et/ou au retrait) et aux supports poreux car ces substrats sont appliqués sur l'adhésif sans passage des substrats dans un four de réticulation et sans doublage des substrats sur un adhésif encore liquide.

Cependant, étant donnée la très faible énergie de surface des adhésifs de silicone, un tel procédé n'est pas applicable (ou applicable de façon très limitée à certains substrats) car la force d'adhésion entre les substrats et le silicone à l'état réticulé est très faible même si le substrat a fait l'objet d'un traitement corona au préalable.

Il en résulte que les produits obtenus :
- ne sont pas dans la grande majorité industriellement faisables : l'adhésion entre l'adhésif silicone et le liner process release est supérieure à l'adhésion entre l'adhésif silicone et le substrat de destination, ce qui implique que le transfert de la masse adhésive ne se produit pas ; et
- peuvent, dans certains cas, être réalisables du point de vue industriel mais présenter des faiblesses rédhibitoires si l'adhésion entre l'adhésif silicone et la peau du patient vient à être supérieure à l'adhésion entre l'adhésif silicone et le substrat de destination, ce qui implique un dysfonctionnement du dispositif médical et/ou des résidus inacceptables d'adhésif sur la peau du patient.

Par ailleurs, dans le domaine industriel général et plus particulièrement dans l'industrie du pansement et des dispositifs médicaux, l'adhésion insuffisante entre un adhésif donnée et un matériau devant être collé peut être renforcée par traitement corona du matériau à coller.

Ainsi par exemple, lors de la dépose de compresse sur un pansement à base d'un support revêtu d'adhésif acrylique, une meilleure tenue de la compresse (en l'état sec ou gorgée d'eau) est obtenue vie traitement corona de la face de la compresse destinée au collage.

Ceci fonctionne très bien pour l'adhésif acrylique et un grand nombre de matériaux à assemble ou coller. Mais pas dans le cas d'un adhésif silicone pour lequel un traitement corona appliqué aux matériaux à assembler ou coller ne donne pas de résultats satisfaisants à cause de la faible énergie de surface dudit adhésif silicone.

Par exemple, dans le cas d'une compresse absorbante de pansement, une faible adhésion sera obtenue lorsque la compresse sera saturée d'exsudats aqueux de la plaie. Le silicone étant hydrophobe par nature, il n'y aura plus d'adhésion entre la compresse et l'adhésif. Le retrait du pansement s'en trouvera alors difficile car les couches du pansement se sépareront les unes des autres.

Le document WO2005/102403 décrit un procédé de préparation d'un matériau adhésif de silicone pour application médicale comprenant une étape de traitement corona sur le substrat destiné à favoriser l'adhésion entre substrat et adhésif.

Ainsi, tous les procédés et matériaux connus et décrits précédemment présentent des limites.

Un but de la présente invention est donc de résoudre les problèmes cités précédemment, à l'aide d'une solution simple à mettre en oeuvre, peu coûteuse, et optimisée en termes d'efficacité, de fiabilité, de durabilité et de qualité du résultat obtenue.

Ainsi, la présente invention a pour objet un procédé de réalisation d'un matériau adhésif pour application médicale tel qu'un pansement, comprenant au moins une étape de revêtement d'une première surface d'un substrat quelconque appelé substrat de destination à l'aide d'une couche de silicone préalablement enduite sur un liner anti-adhérent, caractérisé en ce que, la couche de silicone étant un gel adhésif, le procédé comporte, préalablement à l'étape de revêtement, une étape de traitement corona de la surface du gel silicone adhésif qui est destinée à revêtir le substrat de destination.

L'innovation consiste en l'application du traitement corona appliqué directement sur un adhésif de gel de silicone de façon à en renforcer l'adhésion sur divers substrat pour réaliser des matériaux obtenus par transfert de masse adhésive destinés aux applications médicales.

Selon des modes de réalisation préférés, le dispositif conforme à la présente invention comprend l'une au moins des caractéristiques suivantes :
- le gel de silicone est réticulé avant le traitement corona ;
- l'étape de revêtement du substrat de destination par le gel de silicone adhésif est réalisé en ligne avec l'enduction en une seule étape ;
- l'étape de revêtement du substrat de destination par le gel de silicone adhésif est réalisé hors ligne d'enduction à l'aide d'une étape complémentaire ;
- l'étape de revêtement du substrat de destination par le gel de silicone adhésif est réalisé par enduction continue en pleine surface ;
- le revêtement de gel de silicone adhésif est effectué de manière discontinue de façon à générer des zones localisés dépourvues de gel ;
- le substrat de destination du gel de silicone adhésif est constitué d'un film de matériau synthétique ;
- le substrat de destination du gel de silicone adhésif est constitué d'un textile tissé ou tricoté à partir de fibres ou d'alliages de fibres naturelles et/ou synthétiques ;
- le substrat de destination du gel de silicone adhésif est constitué d'un textile non tissé à partir de fibres ou d'alliages de fibres naturelles et/ou synthétiques, obtenu par le procédé applicable à sa composition, par exemple par voie sèche ou voie humide ;
- le substrat de destination du gel de silicone adhésif est constitué d'une mousse absorbante d'eau ou drainante ayant pour fonction d'absorber ou de drainer les exsudats d'une plaie ;
- le substrat de destination du gel de silicone adhésif est constitué d'une mousse d'amortissement mécanique ayant pour fonction de protéger de chocs, de pression et/ou de frictions ;
- le substrat de destination du gel de silicone adhésif est constitué d'un textile tricoté en trois dimensions à partir de fibres ou d'alliages de fibres naturelles et/ou synthétiques, ayant des propriétés d'amortissement mécanique pour protéger de chocs, de pression et/ou de frictions ;
- le film de matériau synthétique, ou le textile tissé ou tricoté, ou le textile non tissé, ou la mousse absorbante d'eau, ou la mousse d'amortissement mécanique, ou le textile tricoté en trois dimensions est associé(e) à différentes couches fonctionnelles et/ou comporte des additifs fonctionnels tels que des agent d'absorption d'eau ou des agents désinfectants ; et
- le gel de silicone adhésif est teinté dans la masse par des pigments compatibles avec l'application médicale prévue et la composition dudit du gel de silicone adhésif.

L'invention a également pour objet un matériau adhésif pour applications médicales, par exemple pour la réalisation de pansements, obtenu en particulier à l'aide du procédé tel que décrit précédemment, comportant à cet effet un substrat de destination dont une surface est revêtue d'une couche de silicone préalablement enduite sur un liner anti-adhérent, caractérisé en ce que la couche de silicone est un gel de silicone adhésif réticulé et traité corona sur la surface destinée à revêtir le substrat de destination

L'invention va maintenant être décrite plus en détail en référence à des modes de réalisation particuliers donnés à titre d'illustration uniquement et représentés sur les figures annexées dans lesquelles :
- Les figures 1 et 2 sont des schémas illustrant deux procédés de réalisation du matériau conformes à la présente invention ;
- Les figures 3 et 4 sont des vues en coupe illustrant deux étapes de réalisation du matériau ;
- La figure 5 est une vue en coupe d'une variante de réalisation de la figure 4 ;
- La figures 6 est une vue de face de la figure 5 ; et
- Les figures 7 à 10 sont des variantes de réalisation de la figure 6 ;
- La figure 11 est une dernière variante de réalisation de la figure 4.

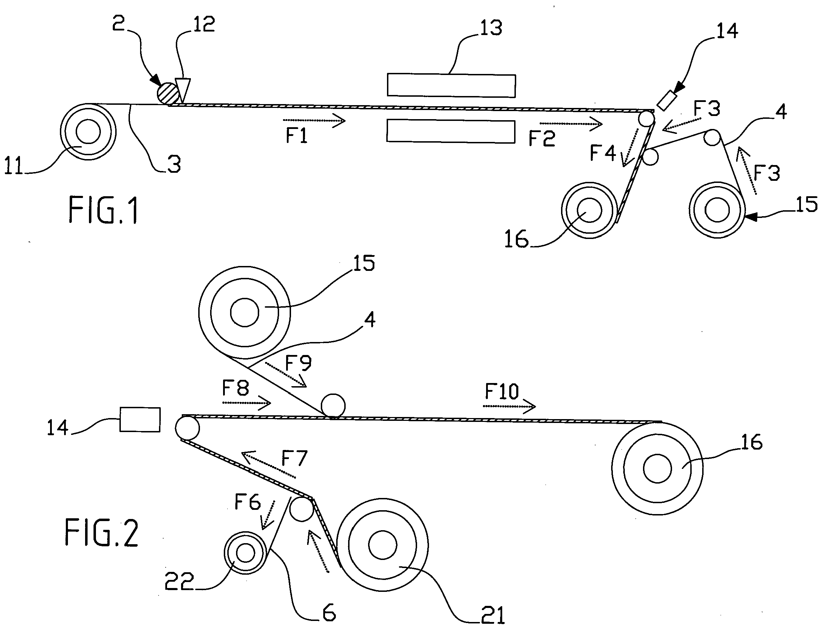

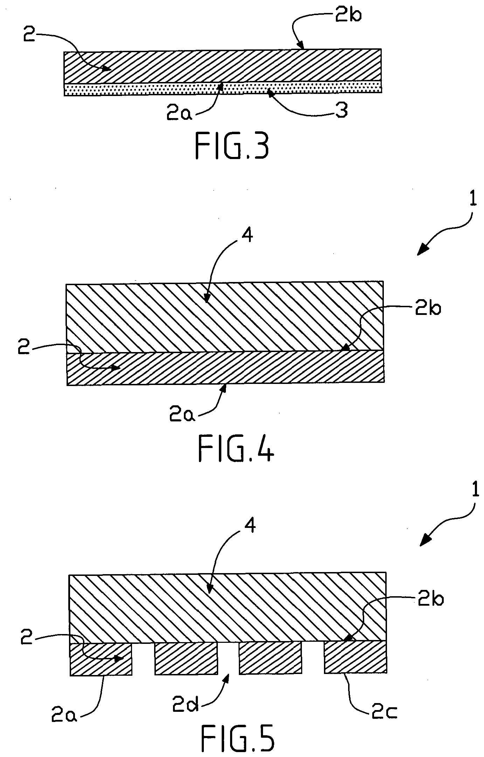

La figure 1 représente un premier mode de réalisation d'un matériau adhésif pour application médicale 1 tel qu'un pansement, comme celui illustré par exemple sur la figure 4.

Dans un premier temps, du gel de silicone adhésif 2 est enduit sur un liner process anti-adhérent 3 en matériau compatible avec le silicone, par exemple du PET, enroulé autour d'une bobine 11. A cet effet, un enducteur transversal 12 (par exemple une racle, des cylindres de transfert, une filière plate d'extrusion ou des buses de dépose de cordon) permet d'étaler le gel de silicone adhésif 2 sur le liner process anti-adhérent 3. Cette opération se déroule en continue, l'ensemble liner/gel de silicone progressant selon la flèche F1.

Dans un deuxième temps, le liner process anti-adhérent 3 enduit de la couche de gel de silicone adhésif 2 traverse un four 13 au sein duquel la couche de gel de silicone adhésif 2 est réticulée sur le liner process anti-adhérent 3. L'ensemble liner/gel de silicone réticulé (voir figure 3) continue sa progression selon la flèche F2.

Dans un troisième temps, le liner process anti-adhérent 3 muni du gel de silicone adhésif réticulé 2 passe dans une unité de traitement corona 14 afin de soumettre la couche de gel de silicone adhésif 2, en particulier sa surface externe 2b (c'est-à-dire la surface opposée au liner process 3), à un traitement corona de type connu.

A cet effet, il convient de donner quelques précisions techniques.

Le gel adhésif de silicone consiste en un polymère de silicone de type PDMS, réticulé et sous forme de gel, obtenu à partir de la réaction chimique de polyaddition entre polyméthylvinylsiloxanes et polyméthylhydrogenosiloxanes en présence de chaleur et d'un catalyseur dérivé du platine.

A la différence du silicone de type élastomère qui présente une dureté élevée, une bonne élasticité, une faible mobilité des chaînes et une surface naturellement non collante (cette dernière propriété est d'ailleurs particulièrement recherchée, utilisée et appréciée dans le domaine concerné), le silicone sous forme de gel présente une très faible dureté, une viscosité élevée, une grande mobilité des chaînes et d'importantes propriétés adhésives.

De manière plus précise, après analyse physicochimique, il s'avère que le gel de silicone traité corona subi les modifications suivantes :
- Création de radicaux libres : CH3 → CH2.
- Oxydation de surface et création de groupements polaires tels qu'hydroxyles et carbonyles.
- Scission du squelette principale de la chaine PDMS -Si-O-Si.
- D'hydrophobe, le silicone devient hydrophile.

En termes de propriétés de surface, les modifications chimiques de surface que subi le gel de silicone réticulé 2 lors du traitement corona se traduisent par une augmentation de l'énergie de surface, évaluée au travers de la mesure de l'angle de contact formé par une goutte d'eau déposée sur la surface du matériau.

La demanderesse a ainsi constaté que la solution innovante de traitement corona du gel de silicone adhésif permet de générer une bonne adhésion sur un film de polyuréthane et ce de façon durable dans le temps.

Une fois le traitement corona réalisé, un substrat de destination 4, par exemple un film de polyuréthane enroulé sur une bobine 15, est immédiatement enduit sur la couche traité corona du gel de silicone adhésif 2 selon les flèches F3 pour une opération dite de doublage et lamination.

Enfin, l'ensemble ainsi formé des trois couches est enroulé sur une bobine 16 selon la flèche F4 pour découpe et utilisation ultérieure, notamment après avoir retiré le liner process anti-adhérent 3 (voir figure 4).

L'innovation permet donc de générer une adhésion forte et durable du gel de silicone adhésif traité corona sur divers substrats qui ne serait pas possible d'obtenir sans traitement.

En effet, après étude, notamment de résistance à l'arrachement, il apparait clairement que la solution classique de traitement corona du substrat utilisée dans le monde des adhésifs acrylique n'a aucun effet dans le cas d'un gel adhésif de silicone alors que la solution innovante de traitement corona du gel adhésif de silicone permet de générer une bonne adhésion sur un film de polyuréthane et ce de façon durable.

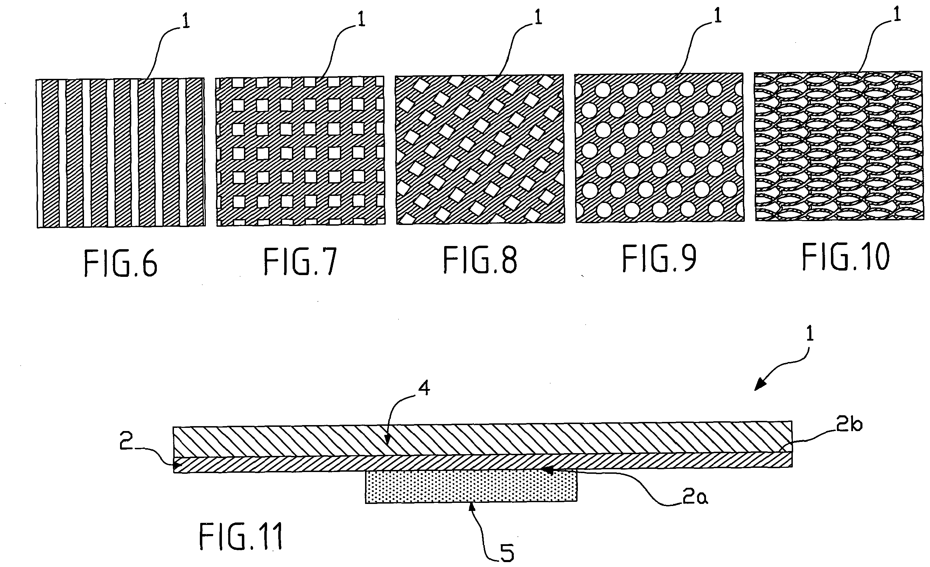

Le procédé de la présente invention permet également de rendre hydrophile la surface du gel de silicone traité corona et donc de conserver l'adhésion entre le gel traité et un substrat hydrophile même gorgé d'eau (voir figure 11 et description associée).

En effet, une deuxième étape de développement a consisté en la comparaison de l'adhésion entre le gel de silicone adhésif et une mousse hydrophile de polyuréthane d'épaisseur 5 mm, et ce pour différentes options de traitement corona, en suivant la performance dans le temps et lorsque la mousse est gorgée d'eau. Comme dans le cas d'un collage sur film polyuréthane, la solution innovante de traitement corona du gel adhésif de silicone permet de générer une bonne adhésion sur une mousse de polyuréthane et ce de façon durable. Par ailleurs, lorsque la mousse est gorgée d'eau, celle-ci reste parfaitement adhérente au gel de silicone.

Le procédé innovant a été appliqué à différents types de matériaux en plus des films et mousses polyuréthanes déjà étudiés, en en particulier :
- un textile extensible tissé à base de fibres de polyamide et de polyuréthane non compatible avec les températures de réticulation du gel de silicone du fait de la présence des fibres de polyuréthane ;
- un textile non tissé par voie sèche par fusion de fibres (meltblown) à base de polyuréthane thermo fusible non compatible avec les températures de réticulation du gel de silicone ;
- un textile non tissé par voie sèche par aiguilletage de fibres (stitchbond) à base fibres de polyester (PET), non compatible avec les températures de réticulation du gel de silicone car présentant un fort retrait thermique et non compatible avec une enduction directe du fait de sa forte porosité ;
- un textile non tissé par voie humide (wetlaid) à base de fibres synthétiques de polyester (PET) et de fibres naturelles de cellulose, compatible avec les températures de réticulation du gel de silicone, mais non compatible avec une enduction directe du fait de sa forte porosité ;
- un film de polyuréthane thermo fusible extrudé (par exemple dont l'une des faces est recouverte d'adhésif acrylique lui-même protégé par un liner anti adhérent.

Pour tous les matériaux réalisés, il a été constaté, un bon démoulage du couple gel/substrat du liner anti adhérent, une bonne aptitude au transfert de masse adhésive du liner vers le substrat et une bonne adhésion entre le gel et le substrat.

Typiquement, le poids de gel adhésif de silicone transféré au substrat est entre 50 et 1000 g/m², de préférence entre 50 et 300 g/m² selon le niveau d'adhésion ciblé sur la peau du patient, défini par l'application finale.

Typiquement, le liner process anti-adhérent compatible avec le gel de silicone adhésif est du type papier ou PET de grammage compris entre 30 et 300 g/m², de préférence entre 40 et 150 g/m², et présentant un revêtement anti-adhérent compatible avec l'adhésif silicone en ce que le silicone peut être enduit sur ce liner, réticulé à son contact et en être ensuite décollé aisément.

La couche de gel est déposée sur le liner anti adhérent par les procédés connus dans l'enduction : racle, par cylindre de transfert, par extrusion au travers d'une filière plate ou par buses de dépose de cordons.

La couche de gel déposée sur le liner est translucide, mais elle peut aussi être teintée dans la masse par des pigments compatibles avec l'application médicale et le système réactif du silicone.

Selon une variante de réalisation illustrée par la figure 2, un produit semi fini, constitué d'un liner process anti-adhérent 3, de gel de silicone adhésif réticulé 2 et d'un liner de protection 6, est fourni à partir d'une bobine 21.

Dans un première temps, le liner de protection 6 est retiré (flèche F6) pour être enroulé sur une bobine 22 tandis que l'ensemble gel de silicone réticulé/liner process poursuit son déplacement selon la flèche F7.

Dans un deuxième temps, le gel de silicone adhésif réticulé 2 est soumis à un traitement corona par l'intermédiaire de l'unité de traitement corona 14 de façon à modifier la structure de la face externe 2b de ladite couche de silicone.

Dans un troisième temps, un substrat 4 est déroulé selon la flèche F8 d'une bobine 15 pour venir enduire la surface 2b traité du gel de silicone adhésif 2 immédiatement après ledit traitement corona de manière à créer une adhérence forte et durable. L'ensemble composé des trois couches (liner process, gel de silicone traité corona et substrat de destination) poursuit son déplacement selon la flèche F10 pour être enroulé sur une bobine de sortie 16 pour découpe et utilisation ultérieure.

Le résultat est identique à celui du procédé de la figure 1. Après avoir retiré le liner process anti-adhérent 3, le matériau adhésif final se présente comme illustré sur la figure 2, avec la surface 2a de silicone adhésif non traité corona destinée à venir au contact de la peau du patient à soigner.

Comme cela est illustré par la figure 5, il est possible d'enduire le liner process anti adhérent 3 de manière discontinue de façon à créer un motif alterné avec des zones pourvues de gel de silicone adhésif 2c et des zones 2d dépourvues de gel de silicone adhésif. Cette solution permet de créer localement une bonne respirabilité à la vapeur d'eau du matériau complexe obtenu.

La figure 6 montre en vue de face le résultat final d'un tel procédé, avec des motifs parallèles en bandes.

D'autres motifs sont possibles tels que les bandes croisées en carrés de la figure 7, les bandes croisées en losanges de la figure 8, les disques de la figure 9 ou les spirales croisées de la figure 10.

Il est également possible de munir le complexe gel de silicone adhésif/substrat de destination d'une compresse 5, comme illustré sur la figure 11. Dans ce cas, une fois le liner process anti-adhérent 3 retiré, la surface 2a de la couche de gel de silicone adhésif 2 est traitée corona de manière à modifier la chimie de surface et l'état de surface du gel préalablement au collage de la compresse 5 pour que cette dernière reste fermement lié audit gel même une humide et imprégnée de sécrétions (par exemple exsudats aqueux provenant d'une plaie).

Il va de soi que la description détaillée de l'objet de l'Invention, donnée uniquement à titre d'illustration, ne constitue en aucune manière une limitation, les équivalents techniques étant également compris dans le champ de la présente invention.

Ainsi, divers films peuvent être utilisés comme substrat de destination, en particulier le polyéthylène (PE), le polypropylène (PP), le polyamide (PA), le Co polyamide (Co PA), le Co polyester (Co PET), le polyuréthane (PU) ou le Co polyuréthane (Co PU).

Concernant les textiles non tissés, des matériaux autres que ceux cités précédemment peuvent être utilisés comme substrat de destination : Cellulose, Viscose.

Concernant les textiles tricotés, il est également possible d'utiliser, en plus de tous les matériaux cités précédemment, l'acétate ou l'acrylique.

Enfin, concernant les compresses, il est possible d'utiliser également les matériaux suivants, avec ou sans agent absorbant tel que carboxy méthyle cellulose, hexa éthyle cellulose, alginate de sodium ou poly acrylate de sodium, avec ou sans agent à caractère désinfectant ou anti microbien, et comprenant ou non un voile anti adhérence sur plaie.

## Revendications

1. Procédé de réalisation d'un matériau adhésif (1) pour application médicale tel qu'un pansement, comprenant au moins une étape de revêtement d'une première surface (2b) d'un substrat quelconque (4 ; 5) appelé substrat de destination à l'aide d'une couche de silicone préalablement enduite sur un liner anti-adhérent (3), **caractérisé en ce que**, la couche de silicone étant un gel adhésif (2), le procédé comporte, préalablement à l'étape de revêtement, une étape de traitement corona de la surface du gel silicone adhésif qui est destinée à revêtir le substrat de destination (4 ; 5).

2. Procédé selon la revendication 1, **caractérisé en ce que** le gel de silicone (2) est réticulé avant le traitement corona.

3. Procédé selon la revendication 2, **caractérisé en ce que** l'étape de revêtement du substrat de destination (4) par le gel de silicone adhésif (2) est réalisé en ligne avec l'enduction en une seule étape.

4. Procédé selon la revendication 2, **caractérisé en ce que** l'étape de revêtement du substrat de destination (4) par le gel de silicone adhésif est réalisé hors ligne d'enduction à l'aide d'une étape complémentaire préalable.

5. Procédé selon l'une quelconque des revendications 2 à 4, **caractérisé en ce que** l'étape de revêtement du substrat de destination (4) par le gel de silicone adhésif (2) est réalisé par enduction continue en pleine surface.

6. Procédé selon l'une quelconque des revendications 2 à 4, **caractérisé en ce que** le revêtement de gel de silicone adhésif (2) est effectué de manière discontinue de façon à générer des zones localisés (2d) dépourvues de gel.

7. Procédé selon l'une quelconque des revendications précédentes, **caractérisé en ce que** le substrat de destination (4) du gel de silicone adhésif (2) est constitué d'un film de matériau synthétique.

8. Procédé selon l'une quelconque des revendications 1 à 6, **caractérisé en ce que** le substrat de destination (4) du gel de silicone adhésif (2) est constitué d'un textile tissé ou tricoté à partir de fibres ou d'alliages de fibres naturelles et/ou synthétiques.

9. Procédé selon l'une quelconque des revendications 1 à 6, **caractérisé en ce que** le substrat de destination (4) du gel de silicone adhésif (2) est constitué d'un textile non tissé à partir de fibres ou d'alliages de fibres naturelles et/ou synthétiques, obtenu par le procédé applicable à sa composition, par exemple par voie sèche ou voie humide.

10. Procédé selon l'une quelconque des revendications 1 à 6, **caractérisé en ce que** le substrat de destination (5) du gel de silicone adhésif (2) est constitué d'une mousse absorbante d'eau ou drainante ayant pour fonction d'absorber ou de drainer les exsudats d'une plaie.

11. Procédé selon l'une quelconque des revendications 1 à 6, **caractérisé en ce que** le substrat de destination (4) du gel de silicone adhésif (2) est constitué d'une mousse d'amortissement mécanique ayant pour fonction de protéger de chocs, de pression et/ou de frictions.

12. Procédé selon l'une quelconque des revendications 1 à 6, **caractérisé en ce que** le substrat de destination (4) du gel de silicone adhésif (2) est constitué d'un textile tricoté en trois dimensions à partir de fibres ou d'alliages de fibres naturelles et/ou synthétiques, ayant des propriétés d'amortissement mécanique pour protéger de chocs, de pression et/ou de frictions.

13. procédé selon l'une quelconque des revendications 7 à 12, **caractérisé en ce que** le film de matériau synthétique, ou le textile tissé ou tricoté, ou le textile non tissé, ou la mousse absorbante d'eau, ou la mousse d'amortissement mécanique, ou le textile tricoté en trois dimensions est associé(e) à différentes couches fonctionnelles et/ou comporte des additifs fonctionnels tels que des agent d'absorption d'eau ou des agents désinfectants.

14. Procédé selon l'une quelconque des revendications précédentes, **caractérisé en ce que** le gel de silicone adhésif (2) est teinté dans la masse par des pigments compatibles avec l'application médicale prévue et la composition dudit du gel de silicone adhésif.

15. Matériau adhésif pour applications médicales (1), par exemple pour la réalisation de pansements, obtenu à l'aide du procédé selon l'une quelconque des revendications précédentes, comportant à cet effet un substrat de destination (4) dont une surface est revêtue d'une couche de silicone préalablement enduite sur un liner anti-adhérent (3), **caractérisé en ce que** la couche de silicone est un gel de silicone adhésif (2) réticulé et traité corona sur la surface (2a, 2b) destinée revêtir le substrat de destination.

## Patentansprüche

1. Verfahren zur Herstellung eines Klebstoffs (1) für medizinische Anwendungen, wie einen Verband, umfassend mindestens einen Schritt des Beschichtens einer ersten Fläche (2b) eines beliebigen Substrats (4; 5), das als Zielsubstrat bezeichnet wird, mit einer Silikonschicht, die zuvor auf einen nicht-haftenden Liner (3) überzogen wurde,
**dadurch gekennzeichnet, dass** die Silikonschicht ein Klebstoffgel (2) ist, wobei das Verfahren, vor dem Beschichtungsschritt, einen Schritt der Corona-Behandlung der Fläche des Klebstoff-Silikongels umfasst, die dazu bestimmt ist, das Zielsubstrat (4; 5) zu beschichten.

2. Verfahren nach Anspruch 1,
**dadurch gekennzeichnet, dass** das Silikongel (2) vor der Corona-Behandlung vernetzt wird.

3. Verfahren nach Anspruch 2,
**dadurch gekennzeichnet, dass** der Schritt des Beschichtens des Zielsubstrats (4) mit dem Klebstoff-Silikongel (2) in-line mit dem Überziehen in einem einzelnen Schritt durchgeführt wird.

4. Verfahren nach Anspruch 2,
**dadurch gekennzeichnet, dass** der Schritt des Beschichtens des Zielsubstrats (4) mit dem Klebstoff-Silikongel off-line mit dem Überziehen mit einem vorherigen ergänzenden Schritt durchgeführt wird.

5. Verfahren nach einem der Ansprüche 2 bis 4,
**dadurch gekennzeichnet, dass** der Schritt des Beschichtens des Zielsubstrats (4) mit dem Klebstoff-Silikongel (2) durch kontinuierliches Überziehen ganzflächig durchgeführt wird.

6. Verfahren nach einem der Ansprüche 2 bis 4,
**dadurch gekennzeichnet, dass** das Beschichten des Klebstoff-Silikongels (2) diskontinuierlich durchgeführt wird, um lokalisierte Zonen (2d) ohne Gel zu erzeugen.

7. Verfahren nach einem der vorhergehenden Ansprüche,
**dadurch gekennzeichnet, dass** das Zielsubstrat (4) des Klebstoff-Silikongels (2) aus einem Film aus synthetischem Material besteht.

8. Verfahren nach einem der Ansprüche 1 bis 6,
**dadurch gekennzeichnet, dass** das Zielsubstrat (4) des Klebstoff-Silikongels (2) aus einem Webstoff oder Gestrick aus Fasern oder aus Legierungen von natürlichen und/oder synthetischen Fasern besteht.

9. Verfahren nach einem der Ansprüche 1 bis 6,
**dadurch gekennzeichnet, dass** das Zielsubstrat (4) des Klebstoff-Silikongels (2) aus einem nicht gewebten Stoff aus Fasern oder aus Legierungen von natürlichen und/oder synthetischen Fasern besteht, die durch das Verfahren erhalten werden, das für seine Zusammensetzung anwendbar ist, beispielsweise auf trockenem oder feuchten Weg.

10. Verfahren nach einem der Ansprüche 1 bis 6,
**dadurch gekennzeichnet, dass** das Zielsubstrat (5) des Klebstoff-Silikongels (2) aus einem wasserabsorbierenden oder ableitenden Schaum mit der Funktion der Absorption oder Drainage von Wundabsonderungen besteht.

11. Verfahren nach einem der Ansprüche 1 bis 6,
**dadurch gekennzeichnet, dass** das Zielsubstrat (4) des Klebstoff-Silikongels (2) aus einem Schaum zur mechanischen Dämpfung mit der Funktion des Schutzes gegen Schläge, Druck und/oder Reibungen besteht.

12. Verfahren nach einem der Ansprüche 1 bis 6,
**dadurch gekennzeichnet, dass** das Zielsubstrat (4) des Klebstoff-Silikongels (2) aus einem dreidimensionalen Gestrick aus Fasern oder aus Legierungen von natürlichen und/oder synthetischen Fasern mit Eigenschaften zur mechanischen Dämpfung besteht, um gegen Schläge, Druck und/oder Reibungen zu schützen.

13. Verfahren nach einem der Ansprüche 7 bis 12,
**dadurch gekennzeichnet, dass** der Film aus synthetischem Material oder der Webstoff oder das Gestrick, oder der nicht gewebte Stoff, oder der wasserabsorbierende Schaum, oder der Schaum zur mechanischen Dämpfung, oder das dreidimensionale Gestrick mit verschiedenen funktionellen Schichten assoziiert ist und/oder funktionelle Additive umfasst, wie Wasserabsorptionsmittels oder Desinfektionsmittel.

14. Verfahren nach einem der vorhergehenden Ansprüche,
**dadurch gekennzeichnet, dass** das Klebstoff-Silikongel (2) in der Masse durch Pigmente gefärbt ist, die mit der vorgesehenen medizinischen Anwendung und der Zusammensetzung des Klebstoff-Silikongels kompatibel sind.

15. Klebstoff (1) für medizinische Anwendungen, beispielsweise zur Herstellung von Verbänden, welcher mit dem Verfahren des Verfahrens nach einem der vorhergehenden Ansprüche erhalten wird, umfassend zu diesem Zweck ein Zielsubstrat (4), von dem eine Fläche mit einer Silikonschicht, die zuvor auf einen nicht-haftenden Liner (3) überzogen wurde, beschichtet ist,
**dadurch gekennzeichnet, dass** die Silikonschicht ein Klebstoff-Silikongel (2) ist, das an der Fläche (2a, 2b), die dazu bestimmt ist, das Zielsubstrat zu beschichten, vernetzt und Coronabehandelt ist.

## Claims

1. Method for producing an adhesive material (1) for a medical application, such as a dressing, comprising at least one step of coating a first surface (2b) of any substrate (4; 5), called the receiving substrate, using a layer of silicone previously applied to an anti-adherent liner (3), **characterised in that**, the silicone layer being an adhesive gel (2), the method comprises, prior to the coating step, a corona treatment step for the surface of the adhesive silicone gel which is intended to coat the receiving substrate (4; 5).

2. Method according to claim 1, **characterised in that** the silicone gel (2) is cross-linked before the corona treatment.

3. Method according to claim 2, **characterised in that** the step of coating the receiving substrate (4) with the adhesive silicone gel (2) is carried out in line with the coating in a single step.

4. Method according to claim 2, **characterised in that** the step of coating the receiving substrate (4) with the adhesive silicone gel is carried out separately from the coating by means of an additional prior step.

5. Method according to any one of claims 2 to 4, **characterised in that** the step of coating the receiving substrate (4) with the adhesive silicone gel (2) is carried out by continuous coating over the entire surface.

6. Method according to any one of claims 2 to 4, **characterised in that** the adhesive silicone gel (2) coating is carried out discontinuously so as to generate localised zones (2d) with no gel.

7. Method according to any one of the preceding claims, **characterised in that** the receiving substrate (4) of the adhesive silicone gel (2) is constituted by a film of synthetic material.

8. Method according to any one of claims 1 to 6, **characterised in that** the receiving substrate (4) of the adhesive silicone gel (2) is constituted by a woven or knitted textile based on natural and/or synthetic fibres or fibre combinations.

9. Method according to any one of claims 1 to 6, **characterised in that** the receiving substrate (4) of the adhesive silicone gel (2) is constituted by a nonwoven textile based on natural and/or synthetic fibres or fibre combinations, obtained using the method applicable to its composition, for example, via the dry or wet route.

10. Method according to any one of claims 1 to 6, **characterised in that** the receiving substrate (5) of the adhesive silicone gel (2) is constituted by a water-absorbent or drainage foam serving to absorb or drain the exudates of a wound.

11. Method according to any one of claims 1 to 6, **characterised in that** the receiving substrate (4) of the adhesive silicone gel (2) is constituted by a mechanical damping foam serving to protect against impacts, pressure and/or friction.

12. Method according to any one of claims 1 to 6, **characterised in that** the receiving substrate (4) of the adhesive silicone gel (2) is constituted by a three-dimensional knitted textile based on natural and/or synthetic fibres or fibre combinations, having mechanical damping properties to protect against impacts, pressure and/or friction.

13. Method according to any one of claims 7 to 12, **characterised in that** the film of synthetic material, or the woven or knitted textile, or the nonwoven textile, or the water-absorbent foam, or the mechanical damping foam, or the three-dimensional knitted textile is associated with different functional layers and/or comprises functional additives, such as water absorption agents or disinfecting agents.

14. Method according to any one of the preceding claims, **characterised in that** the adhesive silicone gel (2) is mass-dyed using pigments compatible with the anticipated medical application and the composition of the adhesive silicone gel.

15. Adhesive material for medical applications (1), for example, for producing dressings, obtained using the method according to any one of the preceding claims, to that end comprising a receiving substrate (4), of which one surface is coated with a layer of silicone previously applied to an anti-adherent liner (3), **characterised in that** the silicone layer is a cross-linked adhesive silicone gel (2) which is subjected to a corona treatment on the surface (2a, 2b) intended to coat the receiving substrate.
